# EUROPEAN PATENT SPECIFICATION

(11) **EP 2 694 471 B1**
(45) Date of publication and mention of the grant of the patent: **02.08.2017**
(21) Application number: 12712124.2
(22) Date of filing: 05.04.2012
(51) Int. Cl.: C07C 303/24, C07C 303/44, G01N 33/78

(54) **PROCESS FOR THE PREPARATION OF A SULFATED DERIVATIVE OF 3,5-DIIODO-O-[3-IODOPHENYL]-L-TYROSINE**
VERFAHREN ZUR HERSTELLUNG EINES SULFATIERTEN DERIVATS VON 3,5-DIIOD-O-[3]-IODOPHENYL]-L-TYROSIN
PROCÉDÉ DE PRÉPARATION D'UN DÉRIVÉ SULFATÉ DE 3,5-DIIODO-O-[3-IODOPHÉNYL]-L-TYROSINE

(30) Priority: 08.04.2011 US 201113083047; 29.04.2011 IT MI20110713
(43) Date of publication of application: 12.02.2014
(73) Proprietor: Bracco Imaging S.p.A., 20134 Milano (IT)
(72) Inventor: ANELLI, Pier Lucio, Colleretto Giacosa, I-1-10010 Ivrea (IT); ARGESE, Maria, I-20018 Sedriano (IT); BOI, Valeria, Colleretto Giacosa, I-1-10010 Ivrea (IT); CAVALIERI, Livio, I-20097 San Donato Milanese (IT); GALIMBERTI, Laura, Colleretto Giacosa, I-1-10010 Ivrea (IT); GAZZETTO, Sonia, Colleretto Giacosa, I-1-10010 Ivrea (IT); LATTUADA, Luciano, Colleretto Giacosa, I-1-10010 Ivrea (IT); MAISANO, Federico, Colleretto Giacosa, I-1-10010 Ivrea (IT); RIVOLTA, Giovanni, I-20097 San Donato Milanese (IT); VELLA, Fulvia, I-20097 San Donato Milanese (IT)
(74) Representative: Merli, Silvia
(86) International application number: PCT/EP2012/056274
(87) International publication number: WO 2012/136761

(56) References cited:
- US-A- 2 970 165
- US-A- 3 313 839
- US-A1- 2005 272 816
- US-A1- 2011 245 342
- JAN A. MOL AND THEO J. VISSER: "Synthesis and Some Properties of Sulfate Esters and Sulfamates of Iodothyronines", ENDOCRINOLOGY, vol. 117, no. 1, 1985, pages 1-7, XP009153115,
- FEIGENBAUM J ET AL: "Simplified Method for the Preparation of Aromatic Sulfuric Acid Esters", JOURNAL OF THE AMERICAN CHEMICAL SOCIETY, AMERICAN CHEMICAL SOCIETY, WASHINGTON, DC; US, vol. 63, 1 January 1941 (1941-01-01), pages 3529-3530, XP002522192, ISSN: 0002-7863, DOI: 10.1021/JA01857A508

## Description

### FIELD OF THE INVENTION

The field of the present invention relates to a process for the preparation of sulfated derivatives of thyroid hormones or salts thereof.

### BACKGROUND OF THE INVENTION

Thyroid hormone tri-iodothyronine (3,5-diiodo-O-[3-iodophenyl]-L-tyrosine or T3) is the metabolically most active thyroid hormone. Like thyroxine (T4) it is physiologically produced by thyroid and stored together with it, under the form of a thyroglobulin, a glycoprotein precursor. On average, one thyroglobulin molecule contains three or four T4 residues and, at the most, one T3 residue. TSH production activates thyroglobulin proteolysis through the enzymes cathepsin D, B and L with the release of thyroid hormones T3 and T4. However, T3 generation is not limited to this mechanism: actually, in the peripheral tissues, thyroxine is transformed into tri-iodothyronine (80% of tri-iodothyronine is periferally produced by thyroxine and 20% is produced inside thyroid gland).

The importance of T3 is not only the one due to the fact of being the most active thyroid hormone. Actually, in this respect, various pathological conditions are known that are caused by its deficiency. In particular, e.g., in nervous tissue during embryonal development and childhood, T3 deficiency gives rise to a reduction in cerebral and cerebellar cortex growth, axons proliferation, cell migration, myelinization, dendrite branching and synapse genesis. As a result of T3 deficiency in the initial stages of life, a delay in the nervous system development is observed followed by a cognitive and motor deficit, that may cause a clinical picture referred to as cretinism. Also in adults it has been demonstrated by cerebral PET that, when the tri-iodothyronine levels are reduced, the blood flow inside the brain and glucose cerebral metabolism are lower. These data may explain the psycomotor deficit in the hypothyroid individuals.

In addition to the effects observed in the nervous tissue, also the ones in the bone tissue are known where the endochondral ossification is stimulated by tri-iodothyronine, thus rendering the bone linearly longer through maturation of the epiphysis bone centers. Even if not necessary after birth for the bone linear growth, tri-iodothyronine is essential for the proper fetus bones development.

Furthermore, T3 effects in the epidermis tissues have been substantiated, where tri-iodothyronine not only takes part in its maturation and of skin adnexa, but also in degradation thereof thus promoting cell regeneration. Therefore, both the excess and the deficiency of this hormone can cause dermatological problems.

Therefore, T3 thyroid hormone may definitely be considered as a pleiotropic hormone, with well documented effects, in addition to the ones above mentioned, in the blood tissue, where it increases erythropoietin production and, consequently, haemopoiesis; in fat tissues, where it promotes maturation of pre-adipocytes to adipocytes, increases the fatty acids lipolysis and finally also regulating cholesterol metabolism.

Hypothyroidism, very frequently generated by autoimmune pathologies, is rather common: actually, prevalence in Italian people is about 1.5% among females and 1% among males. It is pharmacologically treated in a satisfactory way through substitutive therapies, mainly based on synthetic levo-thyroxine (T4), drug of choice because of the very short half-life of the more active form, i.e. T3, which, for this reason, cannot be routinely used.

However, also the therapy with levo-thyroxine shows some disadvantages connected to the fact that while plasmatic euthyroidism is restored, the tissutal one not always does. The study of pharmacological alternatives, such as the ones proposable on the basis of the thyromimetic T3 activity described in EP 1560575 B, might represent a desirable alternative to the present treatments of choice.

However, as far as T3S is involved, the major obstacle seems to be represented by the difficulties met by a large scale synthesis. Actually, until now it has been possible to produce T3S only on a laboratory scale.

In this respect, the preparation of T3S from T3 by means of sulphating agents e.g. concentrated sulphuric acid (H₂SO₄) or chlorosulfonic acid (CSA) in large excess has been described, for example in US2970165 and Biochim. Biophys. Acta, 33, 461 (1959), that describe the preparation of T3S from T3 in solid form, by means of the direct addition of concentrated sulfuric acid, at low temperatures.

Endocrinology, Vol.117, No.1, 1-7 (1985) and Endocrinology, Vol.117, No.1, 8-12 (1985) envisage the synthesis of T3S from T3 by means of the addition under cooling of a chlorosulfonic acid (CSA) solution in dimethylformamide, followed by a purification step through Sephadex LH-20.

Up to now however, none of the prior art processes can be scaled up for grams production of the final product in a pure form, mainly because the reported purification procedures need extremely high volumes.

Advantageously, is has now been found that the sulfation reaction starting from tri-iodothyronine with chlorosulfonic acid (CSA) as a sulfating agent, in the presence of DMAC, offers high conversion rates. Moreover the purification can be carried out with smaller volumes than the ones reported in the known prior-art processes. Eventually, the product T3S can be purified up to the required levels for its clinical use both for the necessary quality and quantity (hundreds of grams), also under conditions applicable on an industrial scale.

### SUMMARY OF THE INVENTION

The present invention relates to a process for the preparation of a mono-cationic salt of 3,5-diiodo-O-[3-iodo-4-(sulfooxy)phenyl]-L-tyrosine of formula II (T3S), by starting from 3,5-diiodo-O-(4-hydroxy-3-iodophenyl)-L-tyrosine of formula I or a salt thereof, according to the scheme: 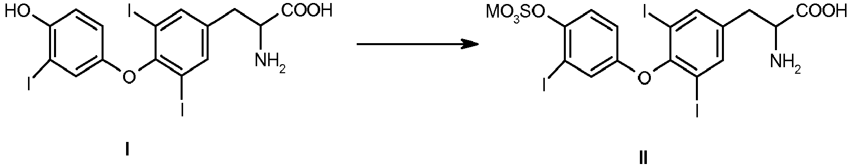 wherein M is an alkali metal, preferably Na,
comprising the steps of:
a) sulfation of the compound of formula I or of the salts thereof with chlorosulfonic acid (CSA) in the presence of dimethylacetamide (DMAC) as a sovent;
b) salification of the sulfated derivative obtained in a) to give the compound of Formula II (T3S) by adding the reaction mixture obtained in a) to an aqueous solution of an alkali metal inorganic salt, preferably a mono-cationic sodium, even more preferably NaHCO₃.

According to a particularly preferred embodiment, the compound of formula I (T3) is obtained by means of the iodination of a compound of formula III (T2): 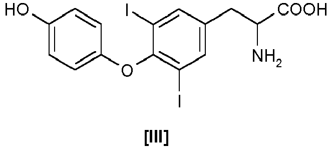 with an iodinating agent, preferably with NaI and I₂, in the presence of an aliphatic amine, preferably selected from linear mono alkyl (C₁-C₄) aliphatic amines, among which, ethylamine is preferred.

The addition of the iodinating agent is carried out in the presence of an aqueous solvent, preferably water, at a temperature preferably lower than 25°C. Preferably, the iodinating agent is present at a molar ratio comprised between 0.9 and 1.1 mol/mol of compound III (T2).

Thus the process for the preparation of T3S comprises the preparation of T3 by means of the iodination of T2 under the conditions above described and then its sulfation with chlorosulfonic acid in dimethylacetamide, as better described in the detailed description.

Moreover, the active principle, T3S, can be formulated into pharmaceutical compositions, preferably solid, wherein T3S, under a powder form, is mixed with a diluting agent and then a flowing agent, a lubricating agent, preferably glicerol dibeenate, and a disaggregating agent, preferably croscaramellose or the derivatives thereof, are added to the mixture their sieving and their further mixing with the diluting mixture comprising the active principle.

Thus according to this aspect, the process comprises a step where the diluent, for example microcrystalline cellulose, is added in one or more fractions, their mixing, then the preparation of a mixture comprising a flowing agent, preferably glicerol dibehenate, a lubricating agent, preferably magnesium or zinc stearate, hydrated colloidal silica, colloidal silicon dioxide and preferably also a disintegrating agent, preferably croscaramellose or the derivatives thereof; then their sieving and their further mixing with the mixture comprising the active principle together with the diluent. Further excipients, stabilizers and diluents (such as for example calcium carbonate) may then be added and mixed for a variable time.

According to a further aspect, a tablet can be prepared by the process above described, comprising T3S as the active principle in a quantity comprised from 1 to 1000 µg and comprising the following diluents, excipients, glidants and lubricants: calcium carbonate, glycerol dibehenate, croscarmellose sodium salt, hydrate colloidal silica, magnesium stearate, microcrystalline cellulose. Quantities for a single dosage are given in the table below:

| | **Amount per Tablet** |
|---|---|
| Calcium carbonate | 20-40 mg |
| Glycerol dibehenate | 2-15 mg |
| Croscarmellose sodium salt | 1-10 mg |
| Hydrate colloidal silica | 0.1-5 mg |
| Magnesium stearate | 0.01-2 mg |
| Microcrystalline cellulose | At least 30 mg |

### DETAILED DESCRIPTION OF THE INVENTION

Object of the present invention is a process for the preparation of a sulfated form of the thyroid hormone T3, 3,5-diiodo-O-[3-iodo-4-(sulfooxy)phenyl]-L-tyrosine (T3S) having formula II as a mono-cationic salt, by starting from 3,5-diiodo-O-[4-hydroxy-3-iodophenyl]-L-tyrosine of formula I or from a salified derivative thereof: 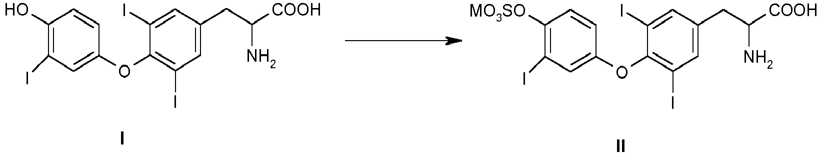 wherein M is an alkali metal, preferably Na, which comprises the steps of:
a) sulfation of the compound of formula I (T3) with chlorosulfonic acid (CSA) in the presence of dimethylacetamide (DMAC) as a sovent,
b) salification of the sulfated derivative obtained in a) to give the compound of formula II. Salification is generally obtained by means of the addition of the reaction mixture obtained in a) to an aqueous solution of an alkali metal inorganic salt, preferably a sodium salt, even more preferably Na₂CO₃ or NaHCO₃.

For the purpose of the present invention by T3S is meant the compound of Formula II comprising either the sulfated form of tri-iodothyronine or the mono-cationic salts thereof (Formula II compound).

Step a) is carried out by adding CSA to a suspension of T3 in DMAC under cooling, while keeping the solution under a vigorous stirring.

Temperature is kept at values lower than about 10°C, more preferably comprised between -10°C and 8°C, more preferably between -8°C and 6°C, even more preferably between -5°C and 5°C.

The addition of CSA to the suspension is made slowly, preferably in a period of time comprised from 30 to 60 min depending on the amount of the reagents employed and preferably under an inert atmosphere, for example under a nitrogen or argon atmosphere.

According to a preferred embodiment, the molar ratio between CSA and T3 is greater than 4, preferably comprised from 4.5 to 10, even more preferably comprised from 7 to 9. Even more preferably comprised from 7.5 to 8.5 mol of CSA/mol of T3. The concentration of T3 in DMAC, expressed as mol of T3/L of DMAC, is comprised from 0.06 to 0.15 mol/L, more preferably from 0.12 to 0.14 mol/L. It follows that, the ratio between CSA and solvent may be comprised from 0.35 to 1.28 mol of CSA/L of DMAC, preferably from about 0.8 to 1.15 mol/L, even more preferably from about 0.96 to 1.1 mol of CSA/L of DMAC.

After adding CSA, the mixture is allowed to react for a period of time not higher than 4-5 hours, generally without cooling, allowing the temperature to reach room temperature (20-25°C).

Sulfation is generally completed, under the described conditions, when more than 85%, preferably more than 90%, even more preferably more than 95% T3 has been converted to T3S.

According to a particularly preferred embodiment, step a) of the process foresees the addition of CSA to a T3 solution in DMAC at a concentration of 0.12-0.14 mol of T3/L of DMAC, with a preferred ratio of about 8 moles of CSA per mole of T3, at a temperature comprised from about -5°C to about 5°C, in a period of time of 30-40 min. At the end of the addition, cooling is generally stopped and the temperature is allowed to rise to room temperature (comprised from about 15 to 25°C), for not more than 4-5 hours, preferably not more than 2-3 hours.

The sulfation mixture is then added according to salification step b), to an aqueous solution of an inorganic alkali salt, preferably mono-cationic, wherein Na is particularly preferred cation, in such an amount as to neutralize the present chlorosulfonic acid.

Salification is preferably carried out with an aqueous solution of sodium carbonate (Na₂CO₃) or sodium hydrogen carbonate (NaHCO₃), in amounts function of the amount of chlorosulfonic acid used in the former step, and at least sufficient to neutralize the pH of the resulting solution. In general, when Na₂CO₃ is used, an amount of about 1.5 moles per mole of CSA is sufficient. According to this embodiment, the Na₂CO₃ solution concentration is about 0.7 mol/L of solution. Under such conditions a solution pH after quenching comprised between 6.5 and 7.5 is obtained.

According to this embodiment, the corresponding mono-cationic salt of the T3S compound obtained, has formula II, wherein M is preferably Na.

The addition of the reaction mixture according to step b) is carried out in a period of time which is variable, typically comprised from 1 h and 3h, while keeping a temperature lower than 30°C.

The T3S compound of formula II, obtained in solution as a mono-cationic salt according to the step b) above described, is purified by chromatography, in accordance to a further step c). Chromatography is previously and optionally preceded by precipitation and/or filtration, for example gravimetrical or under vacuum, of the reaction mixture obtained in b), with the aim of reducing part of the inorganic salts that are formed as by-products.

Chromatography (c) is carried out on an adsorbent resin of the polymer type, a macroreticular aromatic polymeric matrix. Examples of preferred resins are XAD^{™} Amberlites^{™}, even more preferably Amberlite^{™} XAD^{™} 1600.

As well known, before its use, the resin is activated by means of procedures known in the art, such as, for example, washings with water, acetone or the like (for a general reference, see Rohm and Haas in "*Laboratory Column Procedures and Testing of Amberlite and Duolite Polymeric Adsorbents",* section *"Preparation of Resins*"). In accordance with the process of the invention the resin is preferably activated with the solvent selected for the next elution (i.e. acetone or a water/acetone mixture).

T3S is eluted from the resin by an elution mixture of solvents with a decreasing gradient of polarity, starting from the mixture having higher polarity. According to a preferred embodiment, said elution mixture is at first water, followed by successive dilutions with a suitable polar organic solvent, in suitable reciprocal ratios.

Preferred elution mixtures are represented by water/acetonitrile and water/acetone in ratios comprised from 1:0 to 0.7:0.3. Preferably the elution mixture is represented by a mixture of water and acetone in a ratio comprised from 1:0 to 0.85:0.15 and the elution rate through the column is generally comprised from 0.9 to 1.1 volumes of column/h.

The fractions eluted from the column and containing the final product with a purity level higher than 95%, more preferably higher than 96%, 98%, 99% (measured by analytical methods well known in the art, such as for example UV detection and analysed by HPLC analysis) are collected together and the active principle can be isolated by evaporating the solvent, i.e. under vacuum by freeze-drying or by other known methods.

However, according to a preferred embodiment, the eluted fractions are concentrated for example by partial evaporation under vacuum up to a concentration of about 10 g/kg of solution.

At this concentration, the pH of the solution is adjusted to values lower than 6.5, preferably comprised from 5.5 to 6.5, by adding a diluted strong inorganic acid solution, preferably one acid selected between sulfuric acid and hydrochloric acid, being hydrochloric acid particularly preferred, and utilized in diluted form at a concentration comprised from 0.9 to 1.1 N.

The solution is further concentrated about 10-15 times and T3S can be isolated as a solid for example by freeze-drying, spray-drying, or, preferably treated with an organic solvent, preferably of a polar type to be isolated in solid form and then optionally further micronized.

Thus, according to this preferred embodiment, the Formula II compound is isolated in a solid form by treatment with a solvent selected from the group consisting of: acetone, acetonitrile and C₁-C₄ alchools. However other solvents may be employed, which are selected among: aromatic alkanes, ethers, chlorinated solvents, esters, dimethylformamide, nitrometane, dimethylsulfoxide, 2-methoxyethanol, or mixtures thereof, that allow to obtain a salt in solid form, and isolable.

Thus, in detail, after chromatography and concentration of the T3S containing fractions up to a concentration of about 10 g/kg of solution, pH adjustment to values lower then 6.5, preferably comprised from 5.5 to 6.5, and further evaporation up to a concentration of the Formula II compound comprised from 170 to 500 g/kg of suspension or gel, the concentrated solution is treated with an organic solvent. Preferably, said solvent is a polar organic solvent selected among: acetone, lower alchools, such as for example, ethanol, propanol, isopropanol, and the like, and acetonitrile, being acetone particularly preferred.

The addition of acetone to the concentrated T3S solution occurs at a temperature comprised from 20 to 25°C, preferably leaving the mixture under stirring for 1-3 h at a temperature comprised from 0 to 25°C, in order to let the solid form of the mono-cationic T3S salt precipitate completely.

The addition of the solvent to the suspension occurs according to known proportions: when acetone is used, it's added in an amount comprised between 1-11 g acetone/g T3S, at a temperature comprised from 20-25°C.

The mono-cationic derivative of formula II, or more preferably the sodium salt thereof, is thus obtained in solid form after separation of the liquid phase from the solid one, for example by filtration, with a HPLC purity higher than 95%, more preferably, higher than 96%, 98% or even >99%.

Thus, taken as a whole, the process according to the invention allows to obtain isolation of the final product (T3S) in high yields (overall yield: ≥60%) and with a high purity level (HPLC >99%).

Actually, advantageously with prior art processes, already in the sulfation mixture a) in the presence of DMAC, the amount of by-products is lower than 10%, generally lower than 7%.

The high conversion percentage in the sulfation reaction and the following salification allow then to obtain a product in pure form by an industrially applicable chromatographic step and with limited volumes.

T3S is efficiently separated from the other by-products and has high purity (>99%) even when it is prepared in hundreds of grams thus rendering the use of this tri-iodothyronine derivative in clinical practice possible.

In order to prepare formulations for clinical use, T3S, in solid form and with a purity up to 99%, is preferably further micronized, for example under nitrogen pressure, to reduce the particle size.

Particularly preferred is a particle size smaller than 25 µm (at least 90%, more preferably at least 95% of the particles with dimensions lower than 25 µm) resulting stable for at least one month when submitted to accelerated stability trials in a climatic chamber.

Therefore, according to a preferred aspect of the invention, the process comprises micronization of the solid T3S in a pure form, to give particles of the above defined size and the use thereof to prepare solid formulations, for oral administration.

According to this aspect, after micronization, T3S is formulated together with suitable additional components in powder mixtures, optionally also under granular or microgranular form, preferably formulated as tablets or pills obtained through direct compression of the powder mixture.

The T3S formulation in solid form or more preferably into tablets, provides to add, to the micronized active principle (or principles when preferably in combination with levo-thyroxine), firstly a part of the amount of the necessary final diluent, preferably 30, 40, or preferably at least 50% of the diluent, and mixing them to give mixture a).

Preferred diluent is cellulose or the derivatives thereof for example microcrystalline cellulose. Other suitable diluting agents are caolin, starch or alkali inorganic salts such as magnesium or calcium carbonate. Particularly preferred is calcium carbonate, more preferably in association with microcrystalline cellulose.

Mixture a) is then mixed with a mixture b) comprising further components, in general: a glidant agent, a lubricating agent and a disaggregating agent, their sieving and their successive mixing with mixture a) comprising the active principle.

Among the disintegrating agents, particularly preferred is croscaramellose or its derivatives. Other usable agents to this aim are crospovidone, polymethacrylates, maltodestrines, starch sodium glicolate, pre-gelatinized starch, sodium alginate.

Among glidant agents, particularly preferred is glicerol dibehenate. Other usable glidants are: tribasic calcium phosphate, talc, starch or derivatives thereof.

Among the lubricating agents particularly preferred are magnesium or zinc stearate, colloidal hydrated silica, colloidal silicon dioxide. Further excipients, stabilizers and diluents (such as for example calcium carbonate) may be successively added and mixed for a variable time. The final mixture is then measured out and the tablets are preferably prepared by direct compression.

T3S is present in the solid dosage units in amounts comprised from 1 and 1000 µg, more preferably from 2.5 to 500 µg, even more preferably from 5 to 250 µg, as the only active principle, or in combination with other active principles, preferably T4 (levo-thyroxine). According to this embodiment T4 is present in amounts comprised from 1 to 800 µg, or from 5-400 µg, more preferably from 10-200 µg. Accordingly then, in the preparation process of tablets comprising both T3 and T4 as active principles, these are mixed with the preferred diluent(s) in mixture a) and further mixed with the other components, in their turn pre-mixed, as above described.

In the process according to the invention all the reagents including T3 (compound of formula I), are commercially available.

However, according to a particularly preferred embodiment, T3 is prepared by iodination of a compound of formula III (3,5-diiodo-thyronine, Levoditi, or T2): 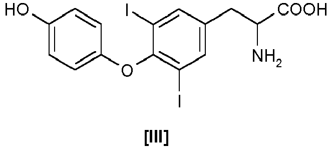 with an iodinating agent, preferably NaI and I₂, in the presence of an aliphatic amine, preferably selected among the mono-alkyl (C₁-C₄) linear aliphatic amines, among which the preferred is ethylamine. T2 is preferably prepared as described.

The addition of the iodinating agent is carried out in the presence of an aqueous solvent, preferably water, at a temperature preferably lower than 25°C.

Preferably the iodinating agent is present at a molar ratio comprised from 0.9 to 1.1 mol/mol of compound III (T2).

After iodination, T3 is isolated, preferably by filtration, as sodium salt, then converted in acid form by re-suspension in water and acidification with an acid, preferably acetic acid or sulfuric acid.

The acid form is isolated, preferably by filtration, again re-suspended in water to remove salts and filtered.

T3, as a wet solid, is suspended in N,N-dimethylacetamide, the suspension is anhydrified and submitted to sulfation reaction.

According to a preferred realization, the molar ratio between CSA and T3 is greater than 4, preferably comprised from 4.5 to 10, even more preferably comprised from 7 to 9. Even more preferably comprised from 7.5 to 8.5 mol of CSA/mol of T3. The concentration of T3 in DMAC, expressed as mol of T3/L of DMAC, is comprised from 0.10 to 0.15 mol/L, more preferably from 0.12 to 0.14 mol/L. It follows that, the ratio between CSA and solvent may be comprised from 0.58 to 1.28 mol of CSA/L of DMAC, preferably from 0.89 to 1.15 mol/L, even more preferably from 0.96 to 1.09 mol of CSA/L of DMAC.

After adding CSA, the mixture is allowed to react for a period of time not higher than 4-5 hours, generally without cooling, allowing the temperature to rise to room temperature.

Sulfation is generally completed, under the described conditions, when more than 85%, preferably more than 90%, even more preferably more than 95% T3 has been converted to T3S.

According to a particularly preferred embodiment, step a) of the process foresees the addition of CSA to a T3 solution in DMAC at a concentration of 0.12-0.14 mol of T3/L of DMAC, with a preferred ratio of about 8 moles of CSA per mole of T3, at a temperature comprised from about -5°C to about 10°C, in a period of time of 30-40 min. At the end of the addition, the cooling is generally stopped and the temperature is allowed to rise to room temperature (comprised from about 15 to 25°C), for not more than 4-5 hours, preferably not more than 2-3 hours.

The sulfation mixture is then added according to salification step b), to an aqueous solution of an inorganic alkali salt, preferably di-cationic, wherein Na is a particularly preferred cation, in such an amount as to neutralize the present chlorosulfonic acid.

Salification is preferably carried out with an aqueous solution of Na₂CO₃ or NaHCO₃, in amounts function of the amount of chlorosulfonic acid used, at least sufficient to neutralize the pH of the resulting solution. In general, when Na₂CO₃ is used, an amount of salt of at least 1.5 moles per mole of CSA is sufficient. When, according to a particularly preferred aspect, the inorganic alkali metal salt is Na₂CO₃, its final concentration is at least 0.7 mol/L solution. Under such conditions, after quenching, a pH of the solution comprised from 6.5 to 7.5 is obtained.

According to this embodiment, the corresponding mono-cationic salt of the T3S compound obtained, has formula II, wherein M is preferably Na.

The addition of the reaction mixture according to step b) is carried out in a period of time which is variable, typically comprised from 1 h and 3h, while keeping a temperature lower than 30°C.

The T3S compound of formula II, obtained in solution as a mono-cationic salt according to steps b) and c) as above described.

The process according to the invention describes for the first time, according to the Applicant's best knowledge, the preparation of T₃S.from either T3 or T2, at a purity of at least 95%, more preferably, of at least 96%, 98% or >99%, for clinical use.

The invention is now described by the following examples which are only explanatory and must not be construed as limitative of the scope of the invention.

### EXPERIMENTAL SECTION

### Example 1. Preparation of T₃S in DMAC

All the amounts of the raw materials are expressed with reference to 100 g of T3.

3,5-diiodo-O-(4-hydroxy-3-iodophenyl)-L-tyrosine (100 g; 0.154 mol) was suspended in DMAC (2.0 L) under nitrogen atmosphere and vigorously stirred in order to avoid solid precipitation. After cooling to -5°C, CSA (142.2 g; 1.229 mol) was added dropwise in 40 min while keeping the temperature between -5 ÷ 5°C. At the end of the addition, cooling was stopped and the reaction mixture was left under stirring for about 4 h. The reaction mixture was added dropwise in 1.5 h, into a stirred aqueous solution of sodium bicarbonate (335.5 g; 3.994 mol in water, 4.5 L). At the end of the addition, from the so obtained solution with time it was observed the precipitation of a crystalline solid as a mixture of inorganic salts. Such a solid was filtered off, then the obtained solution was purified on Amberlite^{™} XAD^{™} 1600 by means of elution with water/acetone mixtures having decreasing polarity collecting the eluate into fractions. The fractions containing the product having an appropriate purity level were evaporated under vacuum up to a concentration of 10 g/kg. The pH of such suspension was adjusted to 6.2 with HCl 1N. The suspension was further concentrated up to a ratio of about 1:3 solid and residual water. Such a residue was treated with acetone under cooling for 2 h, then filtered off and washed with acetone. The product was dried at 40°C under vacuum. 74 g of T₃S were obtained as a white solid. Yield on the anhydrous base: 62%.

### Example 2. Preparation of T₃S from T2 (Levoditi)

The reaction schematic is presented below:

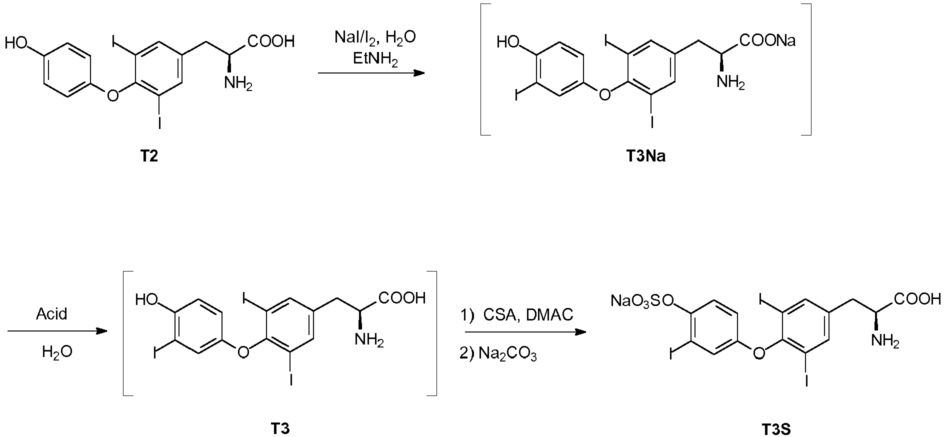

All quantities of raw materials are expressed for 1 kg of Levoditi.

Iodine (approx. 0.48 kg, source: SQM), NaI (approx. 0.65 kg, source: Ajay - SQM) and water were charged in a reactor 18-22°C and stirred until complete dissolution. The resulting iodinating mixture was maintained under stirring at room temperature until use.

Levoditi obtained from L-thyrosine according to the process described in: Chalmers, J. R. et al. A. J. Chem. Soc. 1949, 3424-3433), NaI (approx. 0.32 kg) and water were charged in another reactor and 70% monoethylamine was added.

The iodinating mixture was added to the reaction mixture.

The suspension obtained was stirred for at least 6 h at 18-22°C, then was cooled to 0°C in 1h, stirred for 3-4 h and filtered. The cake was washed with water.

The wet solid was suspended in water and acetic acid was added to the mixture and stirred. The suspension was filtered and the cake washed with water.

The wet solid was re-suspended in water stirred, filtered and washed with water.

The cake was then suspended in DMAC (approx. 12.15 kg) and the suspension was anhydrified distilling under vacuum.

The suspension was cooled to 5-10°C and, in nitrogen atmosphere, CSA (approx. 1.54 kg) was slowly added and the temperature maintained below 15°C.

The solution was heated to 18-22°C in 1 h and maintained for another hour, then was added in a reactor containing a solution of Na₂CO₃ (approx. 2.27 kg) in water (approx. 29.02 kg), previously prepared, maintaining the temperature under 30°C.

The solution was purified onto a column of Amberlite XAD 1600 (12.5 L) by elution of water (87.5 L) and water/acetone mixtures (125 L) with decreasing polarity starting from 95:5 to 70:30. The fractions with high HPLC purity were collected and distilled under vacuum until the desired composition was achieved (approx. 0.04 kg T₃S /L suspension).

The suspension was cooled to 40°C and Ethanol (approx. 5.22 kg) was added, obtaining a solution.

The mixture was cooled to 0°C in 2h, causing precipitation, stirred for another hour and then filtered. The cake was washed with Ethanol/water mixture at room temperature.

Wet solid was dried at approximately 40°C under vacuum.

0.98 kg of pure T3-Sulfate sodium salt (HPLC Area % > 99%) were obtained as a white solid.

Overall yield from T2 (on the anhydrous base): 68.5%.

### Example 3. Preparation of T₃S tablets

The active principle, also in combination with different amounts of levo-thyroxine, was pre-mixed for fifteen minutes with 50% of the content of the microcrystalline cellulose.

To this pre-mixture the following ingredients were added in this order: glicerol dibehenate, colloidal hydrated silica, sodium croscaramellose, magnesium stearate and calcium carbonate (previously sieved through a 0.6 mm clean light/mesh sieve), together with the remaining 50% of the microcrystalline cellulose, mixing for further 20 minutes.

The uniformity of distribution of active principle in the mixture was checked by sampling from six points of the mixer; the text showed that the active principle (or the active principles) uniformly distribute within the mixture, also in the case of formulation with levo-thyroxine.

The powders mixture was then compressed by means of a rotary tablet press equipped with a round flat punch and the tablets were submitted to tests for friability, disaggregation times and the active principle or principles distribution.

The results of the texts performed on the mixing and pressing process confirmed reproducibility of both of them, for T₃S dosages comprised from 25 to 200 µg. Moreover they showed that the tablets so obtained had parameters corresponding to the requirements provided for by the official European Pharmacopoeia (VI Ed.).

**Tablet Composition**

| | |
|---|---|
| T₃S Na salt | 20.6 µg (corresp. to 20 µg T₃S) |
| Calcium carbonate | 30 mg |
| Glycerol dibehenate | 5 mg |
| Croscarmellose sodium salt | 3.5 mg |
| Hydrate colloidal silica | 2 mg |
| Magnesium stearate | 0.5 mg |
| Microcrystalline cellulose | Up to 110 mg |

The tablets prepared as above described were used in clinical trials Phase I on thyroidectomised individuals, showing that they can be used as a thyroid hormone replacement therapy (see US 2011/0245342).

In fact, T₃S was shown to be absorbed (crossing the Gastrointestinal Barrier), was found in serum after oral administration and was converted to the clinically active T3 in a dose-related fashion. T3 levels in serum were still detectable 48 hrs after single dose administration.

### Comparative example: T3S synthesis in DMF and elution trials.

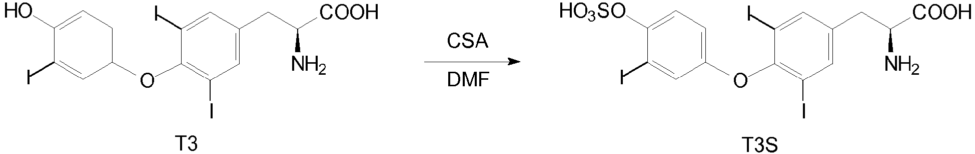

The reaction was carried out according to the scheme above, in DMF. Briefly: T3 (40 mg) was dissolved in ammoniacal ethanol.

This solution was evaporated under a stream of nitrogen.

To the residual, 2 ml of a hot solution of Chlorosulfonic acid (obtained by mixing 2.5 mL of 99% Chlorosulfonic acid and 8 ml of N,N-DMF) was added. Subsequently, the mixture was allowed to reach room temperature under stirring and the reaction was continued overnight.

The mixture was diluted with water (5 mL) and then was eluted on a column of Sephadex LH-20 (5 mL), obtaining fraction A. The elution was continued with 0.1 N HCl (5 mL), obtaining fraction B.

These fractions were re-loaded on column and purified by serial elution of 0.1 N HCl (approx. 4 mL), water and absolute Ethanol.

However, five different water and absolute ethanol quantities were used for purification. The T3S yields and purities obtained by these five conditions have been summarized in Table B.

**Table B: Purification trials**

| **Trial** | **H₂O** | **abs. EtOH** | **T3-Sulfate from aqueous fractions** | | | **T3-Sulfate from alcoholic fractions** | | |
|---|---|---|---|---|---|---|---|---|
| | **(mL)** | **(mL)** | **Amount (mg)** | **Purity^{(a)} (%)** | **Yield (%)** | **Amount (mg)** | **Purity ^{(a)(b)} (%)** | **Yield^{(c)} (%)** |
| 1 | 5 | 10 | 1.0 | 100 | 2.2 | 35 | 80 | 62.3 |
| 2 | 50 | 100 | 2.5 | 100 | 5.6 | 30 | 80 | 53.4 |
| 3 | 125 | 125 | 8.0 | 100 | 17.8 | 30 | 75 | 50.1 |
| 4 | Not registered | 100 | Not registered | Not registered | - | 30 | 75 | 50.1 |
| 5 | 40 | 10 | Not registered | Not registered | - | 10 | 50 | 11.1 |
| | | 20 | Not registered | Not registered | - | 20 | 70 | 31.2 |
| ^{(a) 1}H-NMR purity. | | | | | | | | |
| ^{(b)} From the analyses, the product is a mixture of T3S and T3. | | | | | | | | |
| ^{(c)} Yields are calculated on the content of T3-Sulfate. | | | | | | | | |

Table B shows that when the synthesis is carried out in the conditions described above and DMF is used as the solvent, high conversion may be achieved, but the overall yield is quite low.

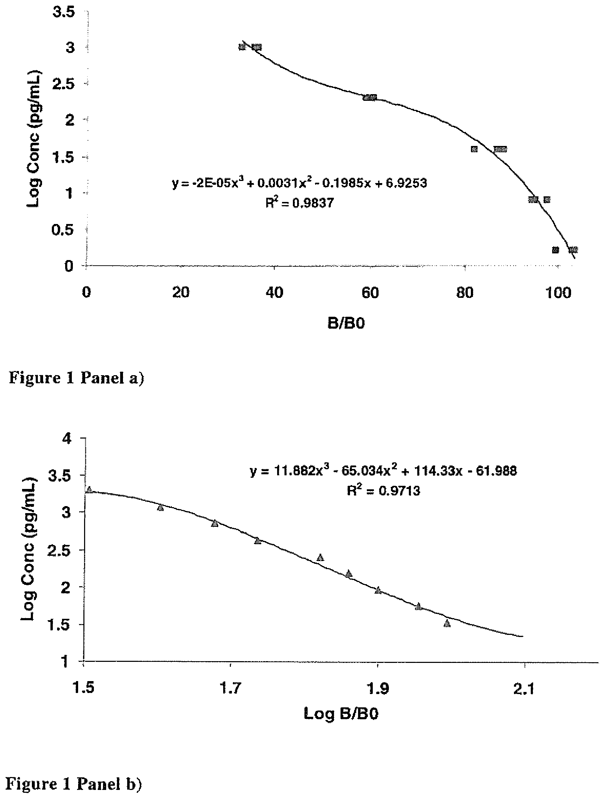

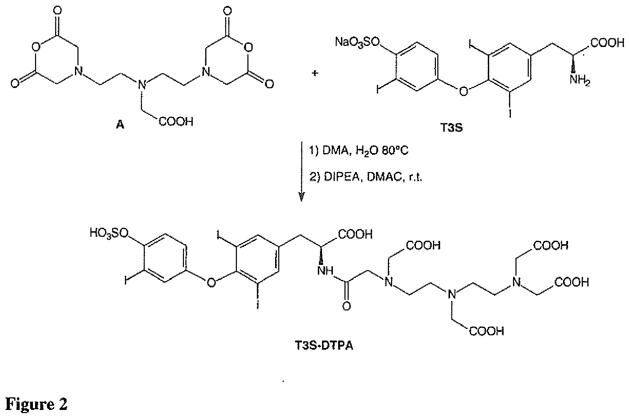

## Claims

1. A process for the preparation of a sulfated form of a thyroid hormone having formula II (T₃S) according to the following reaction: 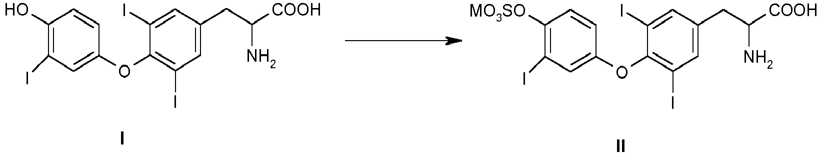 wherein:
M is an alkaline metal, preferably Na;
comprising the steps of:
a) sulfation of a compound of formula I with chlorosulfonic acid (CSA) in the presence of dimethylacetamide (DMAC);
b) salification to give a compound of formula II, in an aqueous solution of an alkaline metal inorganic salt;
c) purification of the formula II compound by chromatography on macroreticular aromatic polymeric matrix and elution with a decreasing polarity mixture of water and an organic solvent.

2. The process according to claim 1, wherein said inorganic salt is a sodium salt.

3. The process according to any one of the claims 1-2, wherein the molar ratio between CSA and the compound of formula I is comprised from 4 to 10, preferably comprised from 7 and 9.

4. The process according to any one of claims 1-3 wherein in step a) the concentration of the compound of formula I in DMAC is comprised from 0.060 and 0.090 mol/L of DMAC.

5. The process according to any one of claims 1-4 wherein the sulfation reaction in step a) is carried out at a temperature below 10° C, preferably comprised from -10° C and 8° C.

6. The process according to claim 5, wherein the sulphatation in step a) is left to occur for at least 2 hours.

7. The process according to any one of claims 1-6, wherein the salification according to step b) is carried out in an aqueous solution of NaHCO₃.

8. The process according to any one of claims 1-7, wherein said step c) is optionally preceded by a filtration step.

9. The process according to any one of claims 1-8, wherein said decreasing polarity mixture is a mixture of water and a polar solvent in a ratio comprised from 1.0:0 and 0.7:0.3.

10. The process according to any one of claims 8-9, wherein after elution, the solution is concentrated up to at least 10g of Formula II compound/kg, and the solution is brought to pH values comprised from 5.5 to 6.5.

11. The process according to any one of claims 1-10, wherein the compound of formula II is obtained as a solid after treatment with an organic polar solvent.

12. The process according to claim 11, wherein said polar organic solvent is selected from: acetone, ethanol, isopropanol and acetonitrile.

13. The process according to any one of claims 11-12, wherein the compound of formula II in the solid form is further micronized.

14. The process according to any one of claims 11-13, which comprises further admixing the solid and/or micronized form of the compound of formula II, optionally in combination with levo-thyroxine (T4), with at least one diluent selected from the group consisting of: cellulose or a derivative thereof, kaolin, starch and inorganic alkaline salts selected from: calcium and magnesium carbonate.

15. The process according to claim 14, wherein the mixture of the compound of formula II and the diluent, wherein the diluent is microcrystalline cellulose, is further admixed with at least one glidant agent selected from the group consisting of: glycerol dibehenate, tribasic calcium phosphate, talc, starch and derivatives thereof, at least one disintegrant selected from the group consisting of: croscarmellose or derivatives thereof, crospovidone, polymethylacrylates, maltodextrin, sodium glycolate starch, pre-gelatinized starch, sodium alginate, and optionally, a lubricating agent selected from the group consisting of: magnesium stearate, zinc stearate, colloidal hydrated silica and colloidal silicon dioxide, and then formulated in tablets by direct compression of the mixture.

16. The process according to any one of claims 1-15, wherein the reagent of formula I is obtained by iodinating a 3',5 di-iodothyronine (T2) with an iodinating agent, preferably a mixture comprising I_{2/}NaI, in an aqueous media and in the presence of an aliphatic amine.

## Patentansprüche

1. Verfahren zur Herstellung einer sulfatierten Form eines Schilddrüsenhormons der Formel II (T₃S) gemäß der folgenden Reaktion: 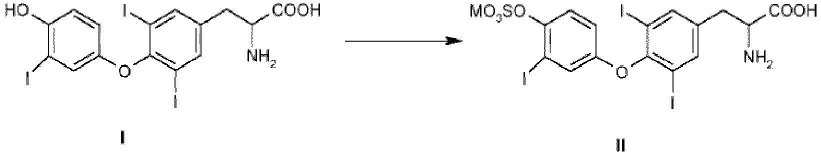 wobei:
M ein Alkalimetall, vorzugsweise Na, bedeutet;
umfassend die folgenden Schritte:
a) Sulfatierung einer Verbindung der Formel I mit Chlorsulfonsäure (CSA) in Gegenwart von Dimethylacetamid (DMAC);
b) Versalzen in einer wässrigen Lösung eines anorganischen Alkalimetallsalzes unter Bildung einer Verbindung der Formel II;
c) Aufreinigung der Verbindung der Formel II mittels Chromatographie an einer makroretikulären aromatischen polymeren Matrix, und Eluieren mit einer aus Wasser und einem organischen Lösungsmittel bestehenden Mischung mit abnehmender Polarität.

2. Verfahren nach Anspruch 1, wobei es sich bei dem anorganischen Salz um ein Natriumsalz handelt.

3. Verfahren nach einem der Ansprüche 1-2, wobei das molare Verhältnis zwischen CSA und der Verbindung der Formel I im Bereich von 4 bis 10, vorzugsweise im Bereich von 7 und 9, liegt.

4. Verfahren nach einem der Ansprüche 1-3, wobei in Schritt a) die Konzentration an Verbindung der Formel I in DMAC im Bereich von 0,060 und 0,090 mol/l DMAC liegt.

5. Verfahren nach einem der Ansprüche 1-4, wobei die Sulfatierung im Schritt a) bei einer Temperatur von unterhalb 10°C, vorzugsweise im Bereich von -10°C und 8°C, liegt.

6. Verfahren nach Anspruch 5, wobei die Sulfatierung im Schritt a) mindestens 2 Stunden lang ablaufen gelassen wird.

7. Verfahren nach einem der Ansprüche 1-6, wobei das Versalzen gemäß Schritt b) in einer wässrigen NaHCO₃-Lösung erfolgt.

8. Verfahren nach einem der Ansprüche 1-7, wobei vor dem Schritt c) gegebenenfalls ein Filtrationsschritt erfolgt.

9. Verfahren nach einem der Ansprüche 1-8, wobei es sich bei der Mischung mit absteigender Polarität um eine Mischung aus Wasser und einem polaren Lösungsmittel in einem Verhältnis im Bereich von 1,0:0 und 0,7:0,3 handelt.

10. Verfahren nach einem der Ansprüche 8-9, wobei nach dem Eluieren die Lösung auf bis zu mindestens 10 g der Verbindung der Formel II/kg eingeengt wird und die Lösung auf pH-Werte im Bereich von 5,5 bis 6,5 eingestellt wird.

11. Verfahren nach einem der Ansprüche 1-10, wobei nach Behandlung mit einem organischen polaren Lösungsmittel die Verbindung der Formel II als Feststoff erhalten wird.

12. Verfahren nach Anspruch 11, wobei das polare organische Lösungsmittel aus Aceton, Ethanol, Isopropanol und Acetonitril ausgewählt ist.

13. Verfahren nach einem der Ansprüche 11-12, wobei die Verbindung der Formel II in fester Form weiterhin mikronisiert wird.

14. Verfahren nach einem der Ansprüche 11-13, das weiterhin das Vermischen der festen und/oder mikronisierten Form der Verbindung der Formel II, gegebenenfalls in Kombination mit levo-Thyroxin (T4), mit mindestens einem Streckmittel, ausgewählt aus der Gruppe bestehend aus Cellulose oder einem Derivat davon, Kaolin, Stärke und anorganischen Alkalisalzen, ausgewählt aus Kalzium- und Magnesiumcarbonat, umfasst.

15. Verfahren nach Anspruch 14, wobei die Mischung der Verbindung der Formel II und des Streckmittels, wobei es sich bei dem Streckmittel um mikrokristalline Cellulose handelt, weiterhin mit mindestens einem Gleitmittel, ausgewählt aus der Gruppe bestehend aus Glycerindibehenat, tribasischem Kalziumphosphat, Talk, Stärke und Derivaten davon, mindestens einem Sprengmittel, ausgewählt aus der Gruppe bestehend aus Croscarmellose oder Derivaten davon, Crospovidon, Polymethylacrylaten, Maltodextrin, Natriumglycolatstärke, vorverkleisterter Stärke, Natriumalginat, und gegebenenfalls einem Schmiermittel, ausgewählt aus der Gruppe bestehend aus Magnesiumstearat, Zinkstearat, kolloidalem hydratisiertem Siliziumdioxid und kolloidalem Siliziumdioxid, vermischt und dann durch direktes Verpressen der Mischung zu Tabletten formuliert wird.

16. Verfahren nach einem der Ansprüche 1-15, wobei das Reagens der Formel I durch Jodieren eines 3',5-Diiodthyronins (T2) mit einem Jodierungsmittel, vorzugsweise einer Mischung umfassend I₂/NaI, in einem wässrigen Medium und in Gegenwart eines aliphatischen Amins erhalten wird.

## Revendications

1. Procédé de préparation d'une forme sulfatée d'une hormone thyroïdienne ayant la formule II (T₃S) selon la réaction suivante : 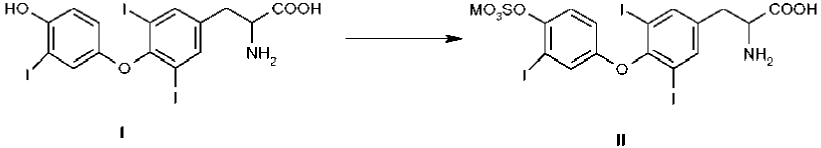 dans laquelle :
M est un métal alcalin, de préférence Na ;
comprenant les étapes de :
a) sulfatation d'un composé de formule I avec l'acide chlorosulfonique (CSA) en présence de diméthylacétamide (DMAC) ;
b) salification pour obtenir un composé de formule II, dans une solution aqueuse d'un sel inorganique de métal alcalin ;
c) purification du composé de formule II par chromatographie sur matrice polymère aromatique macroréticulaire et élution avec un mélange de polarité décroissante d'eau et d'un solvant organique.

2. Procédé selon la revendication 1, dans lequel ledit sel inorganique est un sel de sodium.

3. Procédé selon l'une quelconque des revendications 1 à 2, dans lequel le rapport molaire entre CSA et le composé de formule I est compris entre 4 et 10, de préférence compris entre 7 et 9.

4. Procédé selon l'une quelconque des revendications 1 à 3 dans lequel, dans l'étape a), la concentration du composé de formule I dans DMAC est comprise entre 0,060 et 0,090 mol/l de DMAC.

5. Procédé selon l'une quelconque des revendications 1 à 4 dans lequel la réaction de sulfatation dans l'étape a) est conduite à une température inférieure à 10 °C, de préférence comprise entre -10 °C et 8 °C.

6. Procédé selon la revendication 5, dans lequel la sulfatation dans l'étape a) est conduite pendant au moins 2 heures.

7. Procédé selon l'une quelconque des revendications 1 à 6, dans lequel la salification selon l'étape b) est conduite dans une solution aqueuse de NaHCO₃.

8. Procédé selon l'une quelconque des revendications 1 à 7, dans lequel ladite étape c) est facultativement précédée d'une étape de filtration.

9. Procédé selon l'une quelconque des revendications 1 à 8, dans lequel ledit mélange à polarité décroissante est un mélange d'eau et d'un solvant polaire dans un rapport compris entre 1,0:0 et 0,7:0,3.

10. Procédé selon l'une quelconque des revendications 8 à 9, dans lequel, après élution, la solution est concentrée jusqu'à au moins 10 g de composé de formule II/kg, et la solution est amenée à des valeurs de pH comprises entre 5,5 et 6,5.

11. Procédé selon l'une quelconque des revendications 1 à 10, dans lequel le composé de formule II est obtenu sous la forme d'un solide après traitement avec un solvant polaire organique.

12. Procédé selon la revendication 11, dans lequel ledit solvant polaire organique est choisi parmi : l'acétone, l'éthanol, l'isopropanol et l'acétonitrile.

13. Procédé selon l'une quelconque des revendications 11 à 12, dans lequel le composé de formule II sous la forme solide est en outre micronisé.

14. Procédé selon l'une quelconque des revendications 11 à 13, qui comprend en outre le mélange de la forme solide et/ou micronisée du composé de formule II, facultativement en combinaison avec de la lévo-thyroxine (T4), avec au moins un diluant choisi dans le groupe constitué de : la cellulose ou un dérivé de celle-ci, le kaolin, l'amidon et des sels alcalins inorganiques choisis parmi : le carbonate de calcium et de magnésium.

15. Procédé selon la revendication 14, dans lequel le mélange du composé de formule II et du diluant, le diluant étant la cellulose microcristalline, est en outre mélangé avec au moins un agent glissant choisi dans le groupe constitué des : dibéhénate de glycérol, phosphate de calcium tribasique, talc, amidon et dérivés de celui-ci, au moins un délitant choisi dans le groupe constitué des : croscarmellose ou dérivés de celui-ci, crospovidone, polyméthylacrylates, maltodextrine, glycolate d'amidon sodique, amidon prégélatinisé, alginate de sodium, et facultativement, un agent lubrifiant choisi dans le groupe constitué des : stéarate de magnésium, stéarate de zinc, silice hydratée colloïdale et dioxyde de silicium colloïdal, et ensuite formulé en comprimés par compression directe du mélange.

16. Procédé selon l'une quelconque des revendications 1 à 15, dans lequel le réactif de formule I est obtenu par iodation d'une 3',5-di-iodothyronine (T2) avec un agent d'iodation, de préférence un mélange comprenant I_{2/}NaI, dans un milieu aqueux et en présence d'une amine aliphatique.
